# EUROPEAN PATENT SPECIFICATION

(11) **EP 0 529 883 B1**
(45) Date of publication and mention of the grant of the patent: **22.01.1997**
(21) Application number: 92307400.9
(22) Date of filing: 12.08.1992
(51) Int. Cl.: A61K 7/00, A61K 7/06, A61K 7/48

(54) **Microemulsion**
Mikroemulsion
Microémulsion

(30) Priority: 16.08.1991 GB 9117740
(43) Date of publication of application: 03.03.1993
(73) Proprietor: UNILEVER PLC, London EC4P 4BQ (GB); UNILEVER N.V., 3013 AL Rotterdam (NL)
(72) Inventor: Birtwistle, David Howard, Irby, Wirral L61 9QN (GB)
(74) Representative: Mulder, Cornelis Willem Reinier, Dr.

(56) References cited:
- EP-A- 0 226 337
- EP-A- 0 268 982
- EP-A- 0 432 951
- US-A- 4 733 677

## Description

This invention relates to cosmetic compositions, particularly but not exclusively hair treatment compositions. More specifically, the invention relates to shampoo compositions which include emulsified particles of conditioning oil, which compositions are mechanically stable, preferably optically clear and impart good conditioning benefits to hair and/or skin.

The use of conditioning agents such as silicones in cosmetic formulations, for example hair treatment compositions, is well known and widely documented in the patent literature. Generally, dispersed droplets of the silicone oil are suspended in the composition, which is then applied to the hair to deposit the silicone material on the hair shaft.

Hitherto, steps have had to be taken to prevent the emulsified droplets of silicone oil from agglomerating and the composition creaming during storage. Such steps have for example included the addition of polymers such as Carbopol or certain gums, and/or crystalline materials, e.g. ethylene glycol distearate, to act as suspending agents, but the use of such materials renders the resulting compositions cloudy or opaque. Visually and aesthetically such products are inferior.

The presence of such suspending agents in hair treatment compositions, however, is also disadvantageous because they lead to dulling of the hair, as well as lowering of other conditioning attributes, as a result of the suspending agent being deposited on the hair in addition to the intended silicone conditioning oil.

It is known in the art that oily cosmetic agents such as silicones can be incorporated into cosmetic compositions by means of microemulsification, whereby the silicone is present as stably emulsified droplets of a particle size of the order of 0.15 microns or less.

For example, US 4733677 discloses leave-on hair fixatives containing cationic organic polymer and polydiorganosiloxane microemulsion. EP-A-268982 describes dimethylpolysiloxane microemulsions for various cosmetic uses, the microemulsified dimethylpolysiloxane being formed by emulsion polymerization and with a particle size of 0.15 microns or less.

However, by the very nature of the form in which microemulsified particles of a conditioning oil are incorporated into cosmetic compositions, the conditioning benefits attainable are frequently limited, owing to a poor level of deposition on the intended site, ie. the hair or the skin.

It has now been found that shampoo compositions which have good mechanical stability, high optical transparency or translucency, and excellent conditioning ability can be obtained by utilising a microemulsified conditioning oil in combination with a deposition aid.

Accordingly, in one aspect the present invention provides a shampoo composition comprising:
(a) microemulsified particles of a conditioning oil having a particle size of ≤ 0.15 microns;
(b) a deposition polymer; and
(c) in addition to the microemulsified conditioning oil itself, from about 0.1 to about 50% by weight of at least one surfactant.

The invention will now be described in detail.

As used herein, the term "conditioning oil" includes any material which is used to give a particular conditioning benefit to hair and/or skin. For example, in shampoo compositions for use on the skin, conditioning agents such as moisturizers, essential oils, sun-protective or after-sun treatment materials, occlusive oils and the like may be used. In hair treatment compositions, suitable conditioning agents are those which deliver one or more benefits relating to shine, softness, combability, anti-static properties, wet-handling, damage, stylability and manageability, body and greasiness.

Preferred hair conditioning agents for use in the present invention include volatile and non-volatile silicones, siloxane gums and resins, aminofunctional silicones, quaternary silicones, other oily materials known for use in conditioning hair, and mixtures thereof. Examples of suitable silicone polymers for use in the present invention include those disclosed in EP-A-228575.

Various methods of making microemulsions of particles of conditioning oils for use in the invention are available and are well known and documented in the art.

One particularly preferred technique for making conditioning oil, particularly silicone, microemulsions is that described in EP-A-228575 referred to above. This document described a method of making a stable microemulsion of high molecular weight silicone polymer and water by sequentially adding at an effective rate a standard emulsion comprising polydiorganosiloxane precursor, surfactant and water to a polymerization catalyst medium while mixing to form a clear, stable aqueous microemulsion of polydiorganosiloxane. This emulsion polymerization technique can be used to prepare microemulsions of conditioning agents other than polydiorganosiloxanes for incorporation into compositions of the invention.

Another method of making suitable microemulsions for use in the invention are described in EP-A-0 138 192.

The conditioning oil may for example be a liquid at ambient temperatures, so as to be of a suitable viscosity to enable the material itself to be readily emulsified with the required article size of 0.15 microns or less. However, high viscosity or even solid materials may be appropriate for use in the invention, and indeed may be preferred where in situ polymerisation Is used to prepare the microemulsified particles, as mentioned above. Alternatively, such high viscosity or solid materials may be suitable for use directly if dissolved in a water-immiscible solvent. For example, in the case of a conditioning agent which is a highly viscous silicone resin or siloxane gum, a suitable solvent is a volatile silicone or a volatile hydrocarbon. Examples of all these materials are well documented in the patent literature.

The amount of conditioning oil incorporated into the compositions of the invention may depend on the type of composition and the material used as the conditioning agent. A preferred amount of conditioning oil is from about 0.01 to about 50% by weight of the total composition, more preferably from about 0.01 to about 20% by weight, even more preferably from about 0.01 to about 10% by weight.

The microemulsion of the conditioning agent is stabilised by a suitable amount of one or more emulsifiers, preferably chosen from anionic, cationic, nonionic, amphoteric and zwitterionic surfactants, and mixtures thereof.

Suitable anionic surfactants are the alkyl sulphates, alkyl ether sulphates, alkaryl sulphonates, alkyl succinates, alkyl sulphosuccinates, acyl taurates, acyl glutamates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine and sodium salts of dodecylbenzene sulphonate and sodium N-lauryl sarcosinate. The most preferred anionic surfactants are sodium lauryl ether sulphate 1EO, 2EO, and 3EO, ammonium lauryl sulphate, ammonium lauryl ether sulphate 1EO, 2EO and 3EO, and triethanolamine and sodium salts of dodecylbenzene sulphonate.

Suitable cationic surfactants may include quaternary ammonium hydroxides, e.g. tetramethylammonium hydroxide, octyltrimethylammonium hydroxide, dodecyltrimethylammonium hydroxide, hexadecyltrimethyl-ammonium hydroxide, octyldimethylbenzylammonium hydroxide, decyldimethylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof.

Suitable nonionic surfactants may include condensation products of aliphatic (C₈-C₁₈) primary or secondary linear or branched chain alcohols or phenols with alkylene oxides, usually ethylene oxide and generally having from 6 to 30 ethylene oxide groups.

Other suitable nonionics include alkylpolyglycosides and mono- or di-alkyl alkanolamides. Examples of the latter nonionics include coco mono- or diethanolamide and coco mono-isopropanolamide.

Suitable amphoteric and zwitterionic surfactants may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl and hydroxysultaines, wherein the alkyl and acyl groups have 8 to 19 carbon atoms. Examples include lauryl amine oxide, cocodimethyl sulphopropyl betaine and preferably lauryl betaine, cocamidopropyl betaine and sodium cocamphopropionate.

A particularly preferred shampoo composition in accordance with the invention comprises, in addition to the microemulsified conditioning oil itself, in total from about 1 to about 40% by weight, more preferably from about 2 to about 35% by weight, of at least one surfactant selected from anionic, cationic, nonionic, and amphoteric and zwitterionic surfactants, and mixtures thereof, examples of which are given above.

In accordance with the invention, the shampoo composition contains one or more deposition polymers. Suitable deposition polymers are any which enhance deposition of the conditioning oil on the intended site, i.e. the hair or the skin. A preferred deposition polymer is a cationic deposition polymer, especially a cationic derivative of guar gum or a cationic cellulose ether derivative.

Suitable cationic guar gum derivatives are those given the CTFA designation guar hydroxypropyl trimonium chloride, available commercially for example as JAGUAR C13S, which has a low degree of substitution of the cationic groups and high viscosity. Other suitable materials include that known as JAGUAR C15, having a moderate degree of substitution and a low viscosity, JAGUAR C17 (high degree of substitution, high viscosity) and JAGUAR C16 which is a hydroxypropylated cationic guar derivative containing a low level of substituent groups as well as cationic quaternary ammonium groups. Also suitable is JAGUAR 162 which is a high transparency, medium viscosity guar having a low degree of substitution.

Suitable cationic cellulose ether derivatives are quaternary ammonium derivatives of cellulose ethers, for example the Polymer JR series of materials available from Union Carbide.

The deposition polymer may be present in an amount of from about 0.01 to about 10% by weight of the total composition, preferably from about 0.01 to about 1% by weight, even more preferably from about 0.04 to about 0.5% by weight.

The shampoo compositions of the invention are preferably aqueous based, water forming the basis of the continuous phase of the microemulsion. The compositions preferably comprise water in an amount of from about 20 to about 99% by weight of the total composition.

The compositions of the invention are rinse-off compositions, i.e. suitable for applying to the hair and/or skin, left thereon for an appropriate period of time and then rinsed off with water.

Preferred compositions in accordance with the present invention are optically clear or translucent. However, within the scope of the invention are those compositions as defined which are not themselves transparent or translucent, or which further comprise one or more pearlising and/or opacifying agents.

Depending upon the type of composition and/or conditioning oil employed, one or more additional ingredients conventionally incorporated into cosmetic formulations may be included in the compositions of the invention. Such additional ingredients include suspending agents, opacifiers such as polyethylene glycol distearate, ethylene glycol distearate, polymer latices, antibacterial agents, antidandruff agents, foam boosters, perfumes, colouring agents, preservatives, viscosity modifiers, proteins, polymers, buffering or pH adjusting agents, moisturising agents, herb or other plant extracts and other natural ingredients.

The invention is further illustrated by way of the following non-limitative examples.

### Examples

### Example 1

A hair shampoo composition (A) in accordance with the present invention, comprising microemulsified particles of silicone and a deposition polymer, was prepared as described herein. A similar hair shampoo composition (B) but in accordance with the prior art was also prepared, this composition comprising emulsified silicone of larger particle size and also a suspension system (ethylene glycol distearate). The two compositions had the following formulations:

| Ingredient | (%wt) | |
|---|---|---|
| | A | B |
| SLES 2EO | 16 | 16 |
| Cocoamidopropyl betaine | 2 | 2 |
| Silicone ⁽¹⁾ (particle size 0.036 microns) | 1.0 | - |
| Silicone ⁽²⁾ (particle size 0.38 microns) | - | 1.0 |
| Ethylene glycol distearate | - | 1.5 |
| Jaguar C13S | 0.1 | 0.1 |
| Salt | 1.0 | 1.0 |
| Formalin | 0.1 | 0.1 |
| Water | to 100 | to 100 |

| | | |
|---|---|---|
| ⁽¹⁾ 15,000 cS dimethiconol, added as microemulsion ex Dow Corning. | | |
| ⁽²⁾ 60,000 cS dimethicone, added as emulsion BY22-026 ex Toray Silicone. | | |

Both compositions A and B had a viscosity of approximately 2500 cps. Composition A was transparent, while composition B had a white pearly appearance.

Both compositions were used to treat identical hair switches, which were then subjected to a paired comparison test by trained panellists for various conditioning attributes. The results were as follows:

| | Voting split (maximum 72) | |
|---|---|---|
| | A | B |
| Shine | 51 | 21 |
| Ease of dry combing | 67 | 5 |
| Softness | 68 | 4 |
| Non-flyaway | 56 | 16 |

All of these results are statistically significant in favour of composition A of the invention, with a confidence level of ≥99.9%.

### Example 2

To test the stability of compositions in accordance with the invention, a sample of composition A from Example 1 was compared with a composition corresponding to B in Example 1 but with the ethylene glycol distearate suspending agent omitted.

The latter sample separated, i.e. clear liquid became visible at the bottom of the sample, within 24 hours at 25°C. In contrast, the sample of composition A was stable without visible change for several months both at 25°C and at 45°C.

### Example 3

A series of compositions were prepared to test the level of deposition on hair of microemulsified silicone with and without a deposition polymer, i.e. to compare the level of deposition of compositions in accordance with the invention with similar compositions in accordance with the prior art represented by EP-A-268982. In the following compositions Jaguar C13S and Polymer JR400 are deposition polymers as used in preferred compositions of the invention, whereas Merquat 550 (ex Merck) is a cationic copolymer of dimethyldiaryl-ammonium halide with acrylamide, as disclosed in EP-A-268982 in combination with silicone microemulsion, which latter polymer is understood not to act as a deposition polymer with cosmetic agents.

| Composition (%wt) | | | | | | | | | |
|---|---|---|---|---|---|---|---|---|---|
| Ingredient | 1 | 2 | 3 | 4 | 5 | 6 | 7 | 8 | 9 |
| Sodium lauryl sulphate | 10 | 10 | 10 | - | - | - | - | - | - |
| SLES 2EO | - | - | - | 16 | - | 12 | 12 | 16 | - |
| SLES 3EO | - | - | - | - | 8 | - | - | - | 8 |
| Cocodiethanolamide | 5 | 5 | 5 | - | - | - | - | - | - |
| Cocoamidopropyl betaine | - | - | - | 2 | 4 | 2 | 2 | 2 | 4 |
| Merquat 550⁽⁴⁾ | 1 | 6.3 | - | 1.3 | 3.8 | - | 1.3 | - | - |
| Jaguar C13S⁽⁵⁾ | - | - | 0.5 | - | - | 0.1 | - | 0.1 | - |
| Polymer JR400⁽⁶⁾ | - | - | - | - | - | - | - | - | 0.3 |
| Silicone⁽³⁾ (particle size 0.038 microns) | 1 | 1 | 1 | 1 | 2 | 1 | 1 | 1 | 2 |
| Water | to 100 | to 100 | to 100 | to 100 | to 100 | to 100 | to 100 | to 100 | to 100 |

| | | | | | | | | | |
|---|---|---|---|---|---|---|---|---|---|
| ⁽³⁾ microemulsion as in Example 1 | | | | | | | | | |
| ⁽⁴⁾ contains 8% active | | | | | | | | | |
| ⁽⁵⁾ contains 100% active | | | | | | | | | |
| ⁽⁶⁾ contains 100% active | | | | | | | | | |

Each of the above Compositions 1 to 9 was subjected to a standard deposition test on identical hair switches and the level of silicone deposited in each case was measured using the technique of X-ray fluorescence (XRF), the principles and protocols of which are well described in the scientific literature.

It was observed that with the compositions containing Jaguar C13S and/or Polymer JR400, the level of deposition of silicone was significantly better than the level obtained with the compositions containing Merquat 550.

## Claims

1. A shampoo composition comprising:
(a) microemulsified particles of a conditioning oil having a particle size of ≤0.15 microns;
(b) a deposition polymer; and
(c) in addition to the microemulsified conditioning oil itself, from about 0.1 to about 50% by weight of at least one surfactant.

2. A composition according to claim 1, wherein the conditioning oil is selected from volatile and non-volatile silicones, siloxane gums and resins, aminofunctional silicones, quaternary silicones, and mixtures thereof.

3. A composition according to claim 1 or claim 2, wherein the particles of conditioning oil have a particle size of ≤0.1 microns.

4. A composition according to any one of claims 1 to 3, wherein the microemulsified conditioning oil is present in the composition in an amount of from 0.01 to 50% by weight.

5. A composition according to claim 4, wherein the microemulsified conditioning oil is present in an amount of from 0.01 to 20% by weight of the composition.

6. A composition according to any preceding claim, wherein the deposition polymer is a cationic deposition polymer.

7. A composition according to claim 6, wherein the cationic deposition polymer is a cationic derivative of guar gum or a cationic cellulose ether derivative.

8. A composition according to any preceding claim, wherein the at least one surfactant is selected from anionic, cationic, non-ionic, amphoteric and zwitterionic surfactants, and mixtures thereof.

9. A composition according to any one of claims 1 to 8 which is for treating hair.

10. A composition according to any one of claims 1 to 8 which is for treating skin.

11. A method of conditioning hair or skin comprising applying thereto a shampoo composition which comprises:
(a) microemulsified particles of a conditioning oil having a particle size of ≤0.15 micron s;
(b) a deposition polymer; and
(c) in addition to the microemulsified conditioning oil itself, from about 0.1 to about 50% by weight of at least one surfactant.

12. Use as a hair and/or skin conditioning composition of a microemulsion of a conditioning oil having a particle size of ≤0.15 microns in combination with a deposition polymer.

## Patentansprüche

1. Eine Shampoo-Zusammensetzung, enthaltend:
(a) Mikroemulgierte Teilchen eines Konditionieröls mit einer Teilchengröße von ≤0,15 Mikron;
(b) ein Abscheidungspolymeres; und
(c) außer dem mikroemulgierten Konditionieröl selbst, von etwa 0,1 bis etwa 50 Gewichtsprozent von zumindest einem Surfactant.

2. Eine Zusammensetzung nach Anspruch 1, worin das Konditionieröl aus flüchtigen und nichtflüchtigen Siliconen, Siloxan-Mehlen und Harzen, aminofunktionellen Siliconen, quaternären Siliconen, und Mischungen derselben, ausgewählt ist.

3. Eine Zusammensetzung nach Anspruch 1 oder Anspruch 2, worin die Teilchen des Konditionieröls eine Teilchengröße von ≤0,1 Mikron aufweisen.

4. Eine Zusammensetzung nach einem der Ansprüche 1 bis 3, worin das mikroemulgierte Konditionieröl in der Zusammensetzung in einer Menge im Bereich von 0,01 bis 50 Gewichtsprozent vorhanden ist.

5. Eine Zusammensetzung nach Anspruch 4, worin das mikroemulgierte Konditionieröl in einer Menge im Bereich von 0,01 bis 20 Gewichtsprozent der Zusammensetzung vorhanden ist.

6. Eine Zusammensetzung nach einem der vorstehenden Ansprüche, worin das Abscheidungspolymere ein kationisches Abscheidungspolymeres ist.

7. Eine Zusammensetzung nach Anspruch 6, worin das kationische Abscheidungspolymere ein kationisches Derivat von Guar-Mehl oder einem kationischen Celluloseether-Derivat ist.

8. Eine Zusammensetzung nach einem der vorstehenden Ansprüche, worin das zumindest eine Surfactant aus anionischen, kationischen, nichtionischen, amphoteren und zwitterionischen Surfactants, und Mischungen derselben, ausgewählt ist.

9. Eine Zusammensetzung nach einem der Ansprüche 1 bis 8, welche zur Haarbehandlung dient.

10. Eine Zusammensetzung nach einem der Ansprüche 1 bis 8, welche zur Hautbehandlung dient.

11. Ein Verfahren zum Konditionieren von Haar oder Haut, umfassend das Aufbringen einer Shampoo-Zusammensetzung darauf, welche enthält:
(a) Mikroemulgierte Teilchen eines Konditionieröls mit einer Teilchengröße von ≤0,15 Mikron;
(b) ein Abscheidungspolymeres; und
(c) außer dem mikroemulgierten Konditionieröl selbst, von etwa 0,1 bis etwa 50 Gewichtsprozent von zumindest einem Surfactant.

12. Die Verwendung einer Mikroemulsion eines Konditionieröls mit einer Teilchengröße von ≤0,15 Mikron in Kombination mit einem Abscheidungspolymeren als eine Haar- und/oder Haut-Konditionierzusammensetzung.

## Revendications

1. Composition de shampooing comprenant :
(a) des particules microémulsifiées d'une huile de conditionnement ayant un granulométrie ≤ 0,15 micron ;
(b) un polymère de dépôt ; et
(c) en plus de l'huile de conditionnement microémulsifiée elle-même, de 0,1 à 50% en poids environ d'au moins un tensioactif.

2. Composition selon la revendication 1, dans laquelle on choisit l'huile de conditionnement parmi les silicones volatiles et non volatiles, les gommes et les résines siloxane, les silicones aminofonctionnelles, les silicones quaternaires et leurs mélanges.

3. Composition selon la revendication 1 ou 2, dans laquelle les particules d'huile de conditionnement ont une granulométrie ≤ 0,1 micron.

4. Composition selon l'une quelconque des revendications 1 à 3, dans laquelle l'huile de conditionnement microémulsifiée est présente dans la composition en une quantité de 0,01 à 50% en poids.

5. Composition selon la revendication 4, dans laquelle l'huile de conditionnement microémulsifiée est présente en une quantité de 0,01 à 20% en poids de la composition.

6. Composition selon l'une quelconque des revendications précédentes, dans laquelle le polymère de dépôt est un polymère de dépôt cationique.

7. Composition selon la revendication 6, dans laquelle le polymère de dépôt cationique est un dérivé cationique de gomme de guar ou un dérivé cationique d'éther cellulosique.

8. Composition selon l'une quelconque des revendications précédentes, dans laquelle on choisit le tensioactif parmi les tensioactifs anioniques, cationiques, non ioniques, amphotères et zwitterioniques et leurs mélanges.

9. Composition selon l'une quelconque des revendications 1 à 8, qui est destinée au traitement des cheveux.

10. Composition selon l'une quelconque des revendications 1 à 8, qui est destinée au traitement de la peau.

11. Procédé de conditionnement des cheveux ou de la peau consistant à appliquer une composition de shampooing qui comprend :
(a) des particules microémulsifiées d'une huile de conditionnement ayant un granulométrie ≤ 0,15 micron ;
(b) un polymère de dépôt ; et
(c) en plus de l'huile de conditionnement microémulsifiée elle-même, de 0,1 à 50% en poids environ d'au moins un tensioactif.

12. Utilisation comme composition de conditionnement des cheveux et/ou de la peau d'une microémulsion d'une huile de conditionnement ayant une granulométrie de <0,15 micron en combinaison avec un polymère de dépôt.
